# EUROPÄISCHE PATENTSCHRIFT

(11) **EP 3 747 402 B1**
(45) Veröffentlichungstag und Bekanntmachung des Hinweises auf die Patenterteilung: **20.12.2023**
(21) Anmeldenummer: 20176566.6
(22) Anmeldetag: 26.05.2020
(51) Int. Cl.: A61F 2/16

(54) **INTRAOKULARLINSE**
INTRAOCULAR LENS
LENTILLE INTRAOCULAIRE

(30) Priorität: 06.06.2019 DE 102019115408
(43) Veröffentlichungstag der Anmeldung: 09.12.2020
(73) Patentinhaber: Carl Zeiss Meditec AG, 07745 Jena (DE)
(72) Erfinder: Müller, Marco, 10437 Berlin (DE)
(74) Vertreter: PATERIS Patentanwälte PartmbB

(56) Entgegenhaltungen:
- DE-A1- 10 202 679
- FR-A1- 2 790 661
- FR-B1- 2 790 661

## Beschreibung

Die Erfindung betrifft eine Intraokularlinse.

Bei der Kataraktbehandlung eines Auges wird herkömmlich ein Schnitt in die Hornhaut des Auges eingebracht, der so groß ist, dass eine Kanüle durch den Schnitt in das Auge eingeführt werden kann. Nachdem der Schnitt in die Hornhaut eingebracht wurde, wird die Linse des Auges mittels Phakoemulsifikation zerkleinert und anschließend aus dem Kapselsack des Auges abgesaugt. Danach wird eine Intraokularlinse mittels eines Injektors in den Kapselsack eingesetzt. Die Intraokularlinse weist einen Optikkörper und eine Haptik auf, wobei mittels der Haptik der Optikkörper in dem Kapselsack fixiert wird.

Die Haptik hat die Aufgabe, den Optikkörper möglichst mittig in dem Auge zu halten, um eine möglichst gute Abbildung auf der Netzhaut des Auges zu erzeugen. Zudem soll der Optikkörper möglichst positionsstabil in dem Kapselsack festgelegt sein. Außerdem hat die Haptik die Aufgabe, den Optikkörper davon abzuhalten, eine Rotation um ihreseine optische Achse durchzuführen. Dies ist besonders relevant, wenn es sich bei dem Optikkörper um einen torischen Optikkörper handelt, mittels dessen eine Hornhautverkrümmung der Hornhaut korrigiert werden soll, weil der torische Optikkörper, in dem Fall, dass er mit einer falschen Orientierung in dem Kapselsack angeordnet ist, zu einem Abbildungsfehler auf der Netzhaut führt.

DE 297 10 967 U1 offenbart eine flexible Intra-Okular-Linse aus faltbarem Material, wobei außerhalb eines zentralen Teils mindestens zwei gekrümmte Hebel vorhanden sind, die sich mit etwas größerem Krümmungsradius als der interne Rand des Kapselsacks des Auges an diesen anlegen und über jeweils mindestens zwei Stützarme, die unter einem stumpfen Winkel zum Hebel verlaufen, gehalten werden, so dass sich zwischen den beiden Stützarmen eine Öffnung, ein Fenster, bildet. FR 2 790 661 A1 beschreibt ein intraokulares Augenimplantat. DE 102 02 679 A1 beschreibt eine Intraokularlinse zur Implantation in den Kapselsack eines Auges.

Aufgabe der Erfindung ist es, eine Intraokularlinse zu schaffen, die eine hohe Rotationsstabilität hat.

Die erfindungsgemäße Intraokularlinse weist einen Optikkörper und eine Haptik auf, die ein Gelenkgetriebe aufweist, das einen ersten Arm, der den radial außen angeordneten Teil der Haptik bildet sowie ein erstes Längsende und ein zweites Längsende aufweist, ein erstes Gelenk, das an einer Position zwischen dem ersten Längsende des ersten Arms und dem zweiten Längsende des ersten Arms angeordnet ist und mittels dessen der erste Arm verschwenkbar mit dem Optikkörper gekoppelt ist, einen zweiten Arm, einen dritten Arm, ein zweites Gelenk, ein drittes Gelenk und ein viertes Gelenk aufweist, wobei das zweite Gelenk an dem zweiten Längsende des ersten Arms und an einem ersten Längsende des zweiten Arms angeordnet ist und den ersten Arm verschwenkbar mit dem zweiten Arm koppelt, das dritte Gelenk an einem zweiten Längsende des zweiten Arms und an einem ersten Längsende des dritten Arms angeordnet ist und den zweiten Arm verschwenkbar mit dem dritten Arm koppelt, das vierte Gelenk an einem zweiten Längsende des dritten Arms angeordnet ist und den dritten Arm verschwenkbar mit dem Optikkörper koppelt sowie mindestens eines des zweiten Gelenks, des dritten Gelenks und des vierten Gelenks eingerichtet ist, eine Rückstellkraft auszuüben, wenn das Gelenkgetriebe aus seiner Ruhestellung herausbewegt ist.

Die Intraokularlinse wird mittels eines Injektors in einem gefalteten Zustand der Intraokularlinse in einen Kapselsack eines Auges eingebracht, wobei sich die Intraokularlinse anschließend in dem Kapselsack entfaltet. Weil der erste Arm den radial außen liegenden Teil der Haptik bildet, kontaktiert der erste Arm den Kapselsack entlang einer Kontaktfläche des ersten Arms, nachdem sich die Intraokularlinse entfaltet hat. Zudem drückt der Kapselsack auf den ersten Arm, wobei die Kraft, mittels der der Kapselsack auf den ersten Arm drückt, umso höher ist, je kleiner der Kapselsack ist. Dadurch, dass der Kapselsack auf den ersten Arm drückt, bewegt sich das erste Längsende des ersten Arms in Richtung zu dem Optikkörper hin, wodurch sich das Gelenkgetriebe aus seiner Ruhestellung herausbewegt und damit die Rückstellkraft ausübt. Die Rückstellkraft führt dazu, dass eine Kraft an dem zweiten Längsende des ersten Arms angreift, die zu dem Optikkörper hin gerichtet ist. Weil das erste Gelenk zwischen dem ersten Längsende des ersten Arms und dem zweiten Längsende des ersten Arms angeordnet ist, führt dies dazu, dass an dem ersten Längsende des ersten Arms eine Kraft angreift, die von dem Optikkörper weg gerichtet ist. Damit drückt der erste Arm im Bereich der Kontaktfläche auf den Kapselsack, was dazu führt, dass die Intraokularlinse eine hohe Rotationsstabilität hat. Die hohe Rotationsstabilität ist auch bei verschiedenen Größen der Kapselsäcke gegeben, indem bei einem eher großen Kapselsack sich das erste Längsende des ersten Arms um einen eher kurzen Weg zu dem Optikkörper hin bewegt und bei einem eher kleinen Kapselsack sich das erste Längsende des ersten Arms um einen eher langen Weg zu dem Optikkörper hin bewegt. Die Rückstellkraft steigt linear oder degressiv mit abnehmendem Abstand des ersten Längsendes zu dem Optikkörper an, so dass mit abnehmender Größe des Kapselsacks die Rückstellkraft zunimmt und auch die Rotationsstabilität ansteigt. Bevorzugt weist das Gelenkgetriebe nicht mehr als vier der Gelenke auf. Es ist in Abhängigkeit von dem verwendeten Werkstoff des Gelenkgetriebes auch denkbar, dass die Rückstellkraft mit abnehmendem Abstand des ersten Längsendes zu dem Optikkörper progressiv ansteigt.

Es ist erfindungsgemäß, dass die Haptik einen Abstützvorsprung aufweist, der steifer als das Gelenkgetriebe ausgebildet ist, der an dem Optikkörperbefestigt ist, von dem Optikkörper radial nach außen absteht und an dessen radial äußerem Rand das erste Gelenk angeordnet ist. Somit ist der erste Arm via den Abstützvorsprung verschwenkbar mit dem Optikkörper gekoppelt. Mittels des Abstützvorsprungs lässt sich das erste Gelenk in einem beliebigen Abstand zu dem Optikkörper anordnen. Mittels der Position des ersten Gelenks und des Winkels der Orientierung des ersten Arms relativ zu dem Optikkörper lässt sich einstellen, wie lang der Weg ist, den das erste Längsende des ersten Arms in Richtung zu dem Optikkörper zurücklegen kann.

Der Abstützvorsprung weist mindestens eines der folgenden Merkmale auf, damit der Abstützvorsprung steifer als das Gelenkgetriebe ist:
- der Abstützvorsprung ist in der Richtung parallel zu der optischen Achse des Optikkörpers dicker als das Gelenkgetriebe ausgeführt,
- der Abstützvorsprung weist Rippen auf,
- der Abstützvorsprung ist aus einem Werkstoff, der verschieden von dem Werkstoff des Gelenkgetriebes ist,
- der Abstützvorsprung hat eine stärkere Polymervernetzung als das Gelenkgetriebe,
- der Abstützvorsprung ist faserverstärkt, wobei das Gelenkgetriebe insbesondere nicht faserverstärkt ist. Dabei sind die Rippen bevorzugt in Radialrichtung orientiert oder die Längsrichtung jeder der Rippen schließt an dem Schwerpunkt der jeweiligen Rippe mit der Radialrichtung einen Winkel ein, der nicht größer als 45° ist, insbesondere nicht größer als 30°. Die stärkere Polymervernetzung des Abstützvorsprungs kann dadurch erhalten werden, indem der Abstützvorsprung mit Strahlung, insbesondere UV Strahlung, behandelt wird.

Es ist bevorzugt, dass der Abstützvorsprung eine Aussparung aufweist. Durch das Vorsehen der Aussparung kann der Abstützvorsprung flexibler als ohne die Aussparung gestaltet werden. Bei einem besonders kleinen Kapselsack wird sich zuerst das erste Längsende des ersten Arms um seinen maximal möglichen Weg in Richtung zu dem Optikkörper hin bewegen. Dann wird der Abstützvorsprung komprimiert, was dazu führt, dass auch das Gelenkgetriebe im Bereich des zweiten Arms und dritten Arms komprimiert wird, wodurch an dem zweiten Längsende des ersten Arms eine Kraft angreift, die radial nach außen gerichtet ist und auf den Kapselsack drückt. Diese Kraft führt dazu, dass die Intraokularlinse eine besonders hohe Rotationsstabilität hat. Wenn der Abstützvorsprung komprimiert wird, steigt eine Abstützvorsprungrückstellkraft des Abstützvorsprungs linear oder degressiv oder progressiv mit abnehmendem Abstand des ersten Gelenks zu dem Optikkörper an, so dass mit abnehmender Größe des Kapselsacks die Abstützvorsprungrückstellkraft zunimmt und auch die Rotationsstabilität ansteigt. Es ist besonders bevorzugt, dass die Aussparung ein Durchgangsloch ist, das sich von einem axialen Ende des Abstützvorsprungs bis zu einem anderen axialen Ende des Abstützvorsprungs erstreckt. Mit dem Durchgangsloch kann der Abstützvorsprung besonders flexibel gestaltet werden.

Es ist bevorzugt, dass das mindestens eine des zweiten Gelenks, des dritten Gelenks und des vierten Gelenks ein Filmgelenk ist, um die Rückstellkraft auszuüben. Wie stark die von dem Filmgelenk ausgeübte Rückstellkraft ist, lässt sich über die Stärke des Filmgelenks einstellen, wobei die Stärke des Filmgelenks die Dicke des Filmgelenks in einer Richtung senkrecht zu der optischen Achse ist. Besonders bevorzugt sind das zweite Gelenk, das dritte Gelenk und das vierte Gelenk jeweils eines der Filmgelenke. Ganz besonders bevorzugt hat eines des zweiten Gelenks, des dritten Gelenks und des vierten Gelenks eine höhere Stärke als die anderen beiden Gelenke. Dadurch wird die Rückstellkraft stärker von dem einen Gelenk als von den anderen beiden Gelenken bestimmt. Es ist zudem denkbar, dass die Stärke des einen Gelenks um so viel größer als die Stärke der anderen beiden Gelenke ist, dass die Rückstellkraft im Wesentlichen von dem einen Gelenk bestimmt wird. Es ist zudem bevorzugt, dass das erste Gelenk ebenfalls eines der Filmgelenke ist.

Das Gelenkgetriebe weist bevorzugt einen Vorsprung auf, der an dem ersten Arm befestigt ist und radial nach außen von dem ersten Arm vorsteht. Der Vorsprung kann sich in dem Kapselsack verkrallen und dadurch die Rotationsstabilität der Intraokularlinse erhöhen. Besonders bevorzugt ist der Vorsprung in einem Bereich angeordnet, der sich von dem ersten Gelenk bis zu dem zweiten Längsende des ersten Arms erstreckt. Dadurch bewegt sich der Vorsprung in Richtung von dem Optikkörper weg und zu dem Kapselsack hin, wenn sich das erste Längsende in Richtung zu dem Optikkörper hin bewegt.

Es ist bevorzugt, dass die Haptik in der Richtung parallel zu der optischen Achse des Optikkörpers unterschiedliche Dicken hat. Damit kann die Steifheit der Haptik lokal variiert werden. Es ist besonders bevorzugt, dass in der Richtung parallel zu der optischen Achse des Optikkörpers der erste Arm dicker als die anderen der Arme ausgebildet ist. Dadurch kann der erste Arm formstabil in den Kapselsack eingreifen, wodurch die Intraokularlinse eine besonders hohe Rotationsstabilität hat, während gleichzeitig der zweite Arm und der dritte Arm nachgeben können und dadurch die Verschwenkung des ersten Arms um das erste Gelenk erleichtern. Es ist zudem besonders bevorzugt, dass in der Richtung parallel zu der optischen Achse des Optikkörpers in einem Gelenkbereich, in dem das erste Gelenk angeordnet ist, die Haptik dünner als in den Bereichen der Haptik ist, die an den Gelenkbereich angrenzen. Dadurch wird erreicht, dass das erste Gelenk flexibel ist und somit die Verschwenkung des ersten Arms um das erste Gelenk erleichtert und gleichzeitig der erste Arm formstabil in den Kapselsack eingreifen kann.

Das Gelenkgetriebe weist bevorzugt einen Gelenkgetriebevorsprung auf, der ein erstes Längsende, an dem das vierte Gelenk angeordnet ist, und ein zweites Längsende aufweist und der mit seinem zweiten Längsende an dem Optikkörper befestigt ist. Somit ist der dritte Arm via den Gelenkgetriebevorsprung verschwenkbar mit dem Optikkörper gekoppelt. Dadurch kann das erste Gelenk abseits des Optikkörpers angeordnet werden und stört somit deren optische Funktion nicht.

Es ist bevorzugt, dass in der Ruhestellung das erste Längsende des ersten Arms der am weitesten radial außen angeordnete Teil der Haptik ist.

Die Intraokularlinse weist bevorzugt mindestens zwei der Haptiken auf. Besonders bevorzugt ist, dass jeweils zwei der Haptiken an gegenüberliegenden Enden des Optikkörpers angeordnet sind.

Der Optikkörper ist bevorzugt ein torischer Optikkörper. Die hohe Rotationsstabilität ist besonders bei dem torischen Optikkörper relevant, weil eine Rotation der Intraokularlinse aus ihrer optisch bevorzugten Orientierung heraus zu einem Abbildungsfehler auf der Netzhaut des Auges führt.

Es ist bevorzugt, dass der erste Arm an seiner radial außen angeordneten Seite konvex ausgebildet ist. Dadurch kann die Kontaktfläche, an der der erste Arm den Kapselsack kontaktiert, besonders groß und im Idealfall entlang der gesamten Länge des Arms ausgebildet werden, wodurch die Rotationsstabilität der Intraokularlinse besonders hoch ist. In dem Fall, dass der Abstützvorsprung vorgesehen ist, wird in dem Fall, dass das erste Gelenk eher radial außen vorgesehen ist, die Kontaktfläche eher groß ausgebildet sein und der Weg, den das erste Längsende des ersten Arms in Richtung zu dem Optikkörper zurücklegen kann, eher kurz sein. In dem Fall, dass das erste Gelenk eher radial innen vorgesehen ist, wird die Kontaktfläche eher klein ausgebildet sein und der Weg, den das erste Längsende des ersten Arms in Richtung zu dem Optikkörper zurücklegen kann, eher lang sein.

Im Folgenden wird anhand der beigefügten schematischen Zeichnungen die Erfindung näher erläutert.
Figur 1 zeigt eine erste Ausführungsform der erfindungsgemäßen Intraokularlinse in ihrer Ruhestellung und in einem komprimierten Zustand.
Figur 2 zeigt eine zweite Ausführungsform der erfindungsgemäßen Intraokularlinse in ihrer Ruhestellung und in einem komprimierten Zustand.
Figur 3 zeigt ein Detail der ersten Ausführungsform.
Figur 4 zeigt eine dritte Ausführungsform der erfindungsgemäßen Intraokularlinse in ihrer Ruhestellung.

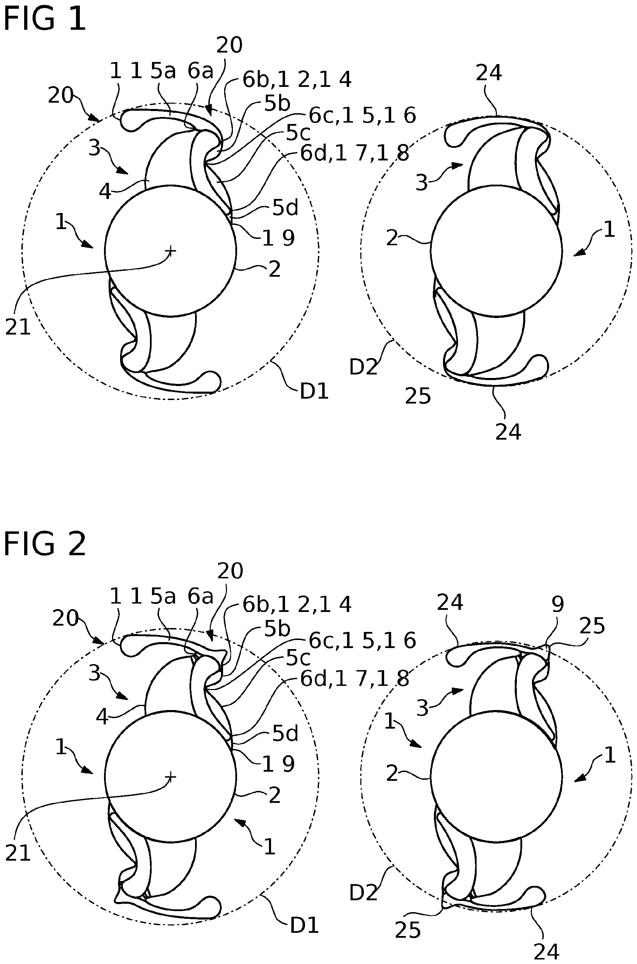

Wie es aus Figuren 1 bis 4 ersichtlich ist, weist eine Intraokularlinse 1 einen Optikkörper 2 und eine Haptik 3 auf. Die Haptik 3 weist ein Gelenkgetriebe 20 auf, das einen ersten Arm 5a, einen zweiten Arm 5b, einen dritten Arm 5c, ein erstes Gelenk 6a, ein zweites Gelenk 6b, ein drittes Gelenk 6c und ein viertes Gelenk 6d aufweist. Denkbar ist, dass das Gelenkgetriebe 20 nicht mehr Gelenke als die vier Gelenke 6a, 6b, 6c, 6d aufweist. Der erste Arm 5a bildet den radial außen angeordneten Teil der Haptik 3 und weist ein erstes Längsende 11 und ein zweites Längsende 12 auf. Das erste Gelenk 6a ist an einer Position zwischen dem ersten Längsende 11 des ersten Arms 5a und dem zweiten Längsende 12 des ersten Arms 5a angeordnet, wobei mittels des ersten Gelenks 6a der erste Arm 5a verschwenkbar mit dem Optikkörper 2 gekoppelt ist. Das zweite Gelenk 6b ist an dem zweiten Längsende 12 des ersten Arms 5a und an einem ersten Längsende 14 des zweiten Arms 5b angeordnet und koppelt den ersten Arm 5a verschwenkbar mit dem zweiten Arm 5b. Das dritte Gelenk 6c ist an einem zweiten Längsende 15 des zweiten Arms 5b und an einem ersten Längsende 16 des dritten Arms 5c angeordnet und koppelt den zweiten Arm 5b verschwenkbar mit dem dritten Arm 5c. Das vierte Gelenk 6d ist an einem zweiten Längsende 17 des dritten Arms 5c angeordnet und koppelt den dritten Arm 5c verschwenkbar mit dem Optikkörper 2. Mindestens eines des zweiten Gelenks 6b, des dritten Gelenks 6c und des vierten Gelenks 6d ist eingerichtet, eine Rückstellkraft auszuüben, wenn das Gelenkgetriebe 20 aus seiner Ruhestellung herausbewegt ist. Es ist denkbar, dass in der Ruhestellung das erste Längsende 11 des ersten Arms 5a der am weitesten radial außen angeordnete Teil der Haptik ist.

Die Ruhestellung, die in Figuren 1 und 2 jeweils in der linken Zeichnung dargestellt ist, zeichnet sich dadurch aus, dass die Haptik 3 im Wesentlichen keine innere mechanische Spannung hat. Der Außendurchmesser der Intraokularlinse 1 erreicht in der Ruhestellung den Wert D1, siehe strichpunktierte Linie in Figur 1 und Figur 2. Wenn das Gelenkgetriebe 20 aus seiner Ruhestellung herausbewegt ist, indem der erste Arm den Kapselsack entlang einer Kontaktfläche 24 des ersten Arms kontaktiert und wie es in Figuren 1 und 2 jeweils in der rechten Zeichnung dargestellt ist, hat sich zuvor der Außendurchmesser zu einem Wert D2 geändert, wobei D1 größer als D2 ist. In diesem Zustand steht das mindestens eine des zweiten Gelenks 6b, des dritten Gelenks 6c und des vierten Gelenks 6d unter einer inneren mechanischen Spannung, welche die Rückstellkraft verursacht. Damit die innere mechanische Spannung entsteht und damit die Rückstellkraft ausgeübt wird, kann das mindestens eine des zweiten Gelenks 6b, des dritten Gelenks 6c und des vierten Gelenks 6d ein Filmgelenk sein. Dabei ist denkbar, dass jedes des zweiten Gelenks 6b, des dritten Gelenks 6c und des vierten Gelenks 6d jeweils eines der Filmgelenke ist. Eines der Filmgelenke kann eine höhere Stärke des Filmgelenks als die anderen beiden der Filmgelenke haben, wobei die Stärke des Filmgelenks die Dicke des Filmgelenks in einer Richtung senkrecht zu der optischen Achse 21 des Optikkörpers 2 ist. Figuren 1, 2 und 4 zeigen, dass dies beispielsweise für das zweite Gelenk 6b der Fall ist, welches eine höhere Stärke als das dritte Gelenk 6c und das vierte Gelenk 6d hat. Figuren 1 bis 4 zeigen, dass es sich bei dem ersten Gelenk 6a ebenfalls um eines der Filmgelenke handeln kann.

Wie es aus Figuren 1, 2 und 4 ersichtlich ist, können das zweite Gelenk 6b, das dritte Gelenk 6c und das vierte Gelenk 6d jeweils C-förmig sein. Dabei können die Öffnungen von zwei benachbarten des zweiten Gelenks 6b, des dritten Gelenks 6c und des vierten Gelenks 6d jeweils einander zugewandt angeordnet sein. Eine solche Konfiguration führt dazu, dass, wenn sich das erste Längsende 11 des ersten Arms 5a aus der Ruhestellung in Richtung zu dem Optikkörper 2 hin bewegt, sich das zweite Gelenk 6b, das dritte Gelenk 6c und das vierte Gelenk 6d aufspreizen.

Figuren 1 bis 4 zeigen, dass das erste Längsende 11 des ersten Arms 5a freigelegt sein kann. Dies bedeutet, dass das erste Längsende 11 des ersten Arms 5a mit keinen anderen Bauteilen der Haptik 3 in Kontakt ist und zwar während der vollständigen Bewegung des ersten Längsendes 11 des ersten Arms 5a aus der Ruhestellung in Richtung zu dem Optikkörper 2 hin.

Gemäß den Figuren 1 bis 4 weist die Haptik 3 einen Abstützvorsprung 4 auf, der steifer als das Gelenkgetriebe 20 ausgebildet ist. Der Abstützvorsprung 4 ist an dem Optikkörper 2 befestigt und steht von dem Optikkörper 2 radial nach außen ab. An dem radial äußeren Rand des Abstützvorsprungs 4 ist das erste Gelenk 6a angeordnet. Der Abstützvorsprung 4 kann mindestens eines der folgenden Merkmale aufweisen, damit der Abstützvorsprung steifer als das Gelenkgetriebe 20 ist:
- Der Abstützvorsprung ist in der Richtung parallel zu der optischen Achse 1 des Optikkörpers 2 dicker als das Gelenkgetriebe 20 ausgeführt.
- Der Abstützvorsprung 4 weist Rippen auf. Die Rippen können sich beispielsweise ausgehend von dem Optikkörper 2 bis zu dem radial äußeren Rand des Abstützbereichs 4 erstrecken. Die Rippen können an beiden axialen Seiten des Abstützvorsprungs 4 oder an nur einer axialen Seite des Abstützvorsprungs 4 vorgesehen sein. Dabei sind die Rippen bevorzugt in Radialrichtung orientiert oder die Längsrichtung jeder der Rippen schließt an dem Schwerpunkt der jeweiligen Rippe mit der Radialrichtung einen Winkel ein, der nicht größer als 45° ist, insbesondere nicht größer als 30°.
- Der Abstützvorsprung 4 ist aus einem Werkstoff, der verschieden von dem Werkstoff des Gelenkgetriebes 20 ist.
- Der Abstützvorsprung 4 hat eine stärkere Polymervernetzung als das Gelenkgetriebe 20. Dies kann beispielsweise erzeugt werden, indem der Abstützvorsprung 4 mit Strahlung, insbesondere UV Strahlung, behandelt wird.
- Der Abstützvorsprung 4 ist faserverstärkt und das Gelenkgetriebe ist nicht faserverstärkt.

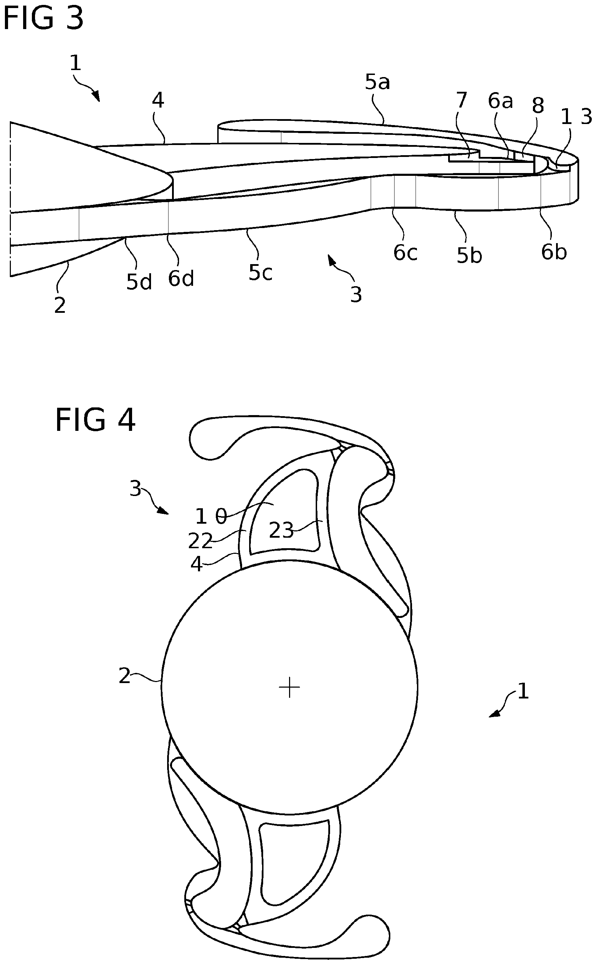

Wie es aus Figur 4 ersichtlich ist, kann der Abstützvorsprung 4 eine Aussparung 10 aufweisen. Bei der Aussparung 10 kann es sich auch um ein Durchgangsloch handeln, das sich von einem axialen Ende des Abstützvorsprungs 4 bis zu dem anderen axialen Ende des Abstützvorsprungs 4 erstreckt. In diesem Fall weist der Abstützvorsprung 4 einen ersten Steg 22 und einen zweiten Steg 23 auf, die sich ausgehend von voneinander beabstandeten Positionen am radial äußeren Ende des Optikkörpers 2 erstrecken und an dem radial äußeren Rand des Abstützbereichs 4 zusammengeführt sind.

Figur 2 zeigt, dass das Gelenkgetriebe 20 einen Vorsprung 9 aufweisen kann, der an dem ersten Arm 5a befestigt ist und radial nach außen von dem ersten Arm 5a vorsteht. An dem Vorsprung 9 kann eine weitere Kontaktfläche 25 ausgebildet sein, an der der Vorsprung 9 den Kapselsack kontaktiert. Der Vorsprung 9 kann, wie es in Figur 2 dargestellt ist, an dem zweiten Längsende 12 des ersten Arms 5a angeordnet sein. Alternativ ist es denkbar, dass der Vorsprung 9 abseits des zweiten Längsendes 12 des ersten Arms 5a in einer Position zwischen dem ersten Gelenk 6a und dem zweiten Längsende 12 des ersten Arms 5a angeordnet ist.

Figur 3 zeigt, dass die Haptik 3 in der Richtung parallel zu der optischen Achse 21 des Optikkörpers 2 unterschiedliche Dicken haben kann. Beispielsweise kann in der Richtung parallel zu der optischen Achse 21 des Optikkörpers 2 in einem Gelenkbereich, in dem das erste Gelenk 6a angeordnet ist, die Haptik 3 dünner als in den Bereichen der Haptik 3 sein, die an den Gelenkbereich angrenzen. Dadurch weist die Haptik 3 eine erste Stufe 7 und eine zweite Stufe 8 auf, wobei die erste Stufe 7 an einem Übergang zwischen dem Abstützvorsprung 4 und dem Gelenkbereich angeordnet ist und die zweite Stufe 8 an einem Übergang zwischen dem Gelenkbereich und dem ersten Arm 5a angeordnet ist. Beispielsweise kann in der Richtung parallel zu der optischen Achse 21 des Optikkörpers 2 der erste Arm 5a dicker als der zweite Arm 5b und der dritte Arm 5c ausgebildet sein. Dadurch weist das Gelenkgetriebe 20 eine dritte Stufe 13 auf, die an dem zweiten Längsende 12 des ersten Arms 5a angeordnet ist.

Wie es aus Figuren 1 bis 4 ersichtlich ist, kann das Gelenkgetriebe 20 einen Gelenkgetriebevorsprung 5d aufweisen, der ein erstes Längsende 18, an dem das vierte Gelenk 6d angeordnet ist, und ein zweites Längsende 19 aufweist, wobei der Gelenkgetriebevorsprung 5d mit seinem zweiten Längsende 19 an dem Optikkörper 2 befestigt ist. Damit ist der dritte Arm 5c via den Gelenkgetriebevorsprung 5d verschwenkbar mit dem Optikkörper 2 gekoppelt.

Der erste Arm 5a kann, wie es in Figuren 1 bis 4 dargestellt ist, an seiner radial außen angeordneten Seite konvex ausgebildet sein.

Bei dem Optikkörper 2 kann es sich um einen torischen Optikkörper handeln.

Die Intraokularlinse 1 kann zwei der Haptiken 3 aufweisen, die an gegenüberliegenden Seiten des Optikkörpers 2 angeordnet sind.

### Bezugszeichenliste

1 Intraokularlinse
2 Optikkörper
3 Haptik
4 Abstützvorsprung
5a erster Arm
5b zweiter Arm
5c dritter Arm
5d Gelenkgetriebevorsprung
6a erstes Gelenk
6b zweites Gelenk
6c drittes Gelenk
6d viertes Gelenk
7 erste Stufe
8 zweite Stufe
9 Vorsprung
10 Aussparung
11 erstes Längsende des ersten Arms
12 zweites Längsende des ersten Arms
13 dritte Stufe
14 erstes Längsende des zweiten Arms
15 zweites Längsende des zweiten Arms
16 erstes Längsende des dritten Arms
17 zweites Längsende des dritten Arms
18 erstes Längsende des Gelenkgetriebevorsprungs
19 zweites Längsende des Gelenkgetriebevorsprungs
20 Gelenkgetriebe
21 optische Achse
22 erster Steg
23 zweiter Steg
24 Kontaktfläche
25 weitere Kontaktfläche
D1 Außendurchmesser der Intraokularlinse in Ruhestellung
D2 Außendurchmesser der Intraokularlinse im komprimierten Zustand

## Patentansprüche

1. Intraokularlinse mit einem Optikkörper (2) und einer Haptik (3), **dadurch gekennzeichnet, dass** die Haptik (3) ein Gelenkgetriebe (20) aufweist, das einen ersten Arm (5a), der den radial außen angeordneten Teil der Haptik (3) bildet sowie ein erstes Längsende (11) und ein zweites Längsende (12) aufweist, ein erstes Gelenk (6a), das an einer Position zwischen dem ersten Längsende (11) des ersten Arms (5a) und dem zweiten Längsende (12) des ersten Arms (5a) angeordnet ist und mittels dessen der erste Arm (5a) verschwenkbar mit dem Optikkörper (2) gekoppelt ist, einen zweiten Arm (5b), einen dritten Arm (5c), ein zweites Gelenk (6b), ein drittes Gelenk (6c) und ein viertes Gelenk (6d) aufweist, wobei das zweite Gelenk (6b) an dem zweiten Längsende (12) des ersten Arms (5a) und an einem ersten Längsende (14) des zweiten Arms (5b) angeordnet ist und den ersten Arm (5a) verschwenkbar mit dem zweiten Arm (5b) koppelt, das dritte Gelenk (6c) an einem zweiten Längsende (15) des zweiten Arms (5b) und an einem ersten Längsende (16) des dritten Arms (5c) angeordnet ist und den zweiten Arm (5b) verschwenkbar mit dem dritten Arm (5c) koppelt, das vierte Gelenk (6d) an einem zweiten Längsende (17) des dritten Arms (5c) angeordnet ist und den dritten Arm (5c) verschwenkbar mit dem Optikkörper (2) koppelt sowie mindestens eines des zweiten Gelenks (6b), des dritten Gelenks (6c) und des vierten Gelenks (6d) eingerichtet ist, eine Rückstellkraft auszuüben, wenn das Gelenkgetriebe (20) aus seiner Ruhestellung herausbewegt ist, wobei die Haptik (3) einen Abstützvorsprung (4) aufweist, der steifer als das Gelenkgetriebe (20) ausgebildet ist, der an dem Optikkörper (2) befestigt ist, von dem Optikkörper nach radial außen absteht und an dessen radial äußerem Rand das erste Gelenk (6a) angeordnet ist.

2. Intraokularlinse gemäß Anspruch 1, wobei der Abstützvorsprung (4) mindestens eines der folgenden Merkmale aufweist, damit der Abstützvorsprung steifer als das Gelenkgetriebe (20) ist:
- der Abstützvorsprung (4) ist in der Richtung parallel zu der optischen Achse (21) des Optikkörpers (2) dicker als das Gelenkgetriebe (20) ausgeführt,
- der Abstützvorsprung (4) weist Rippen auf,
- der Abstützvorsprung (4) ist aus einem Werkstoff, der verschieden von dem Werkstoff des Gelenkgetriebes (20) ist,
- der Abstützvorsprung (4) hat eine stärke Polymervernetzung als das Gelenkgetriebe (20),
- der Abstützvorsprung (4) ist faserverstärkt.

3. Intraokularlinse gemäß Anspruch 1 oder 2, wobei der Abstützvorsprung (4) eine Aussparung (10) aufweist, insbesondere wobei die Aussparung (10) ein Durchgangsloch ist.

4. Intraokularlinse gemäß einem der Ansprüche 1 bis 3, wobei das mindestens eine des zweiten Gelenks (6b), des dritten Gelenks (6c) und des vierten Gelenks (6d) ein Filmgelenk ist, um die Rückstellkraft auszuüben.

5. Intraokularlinse gemäß einem der Ansprüche 1 bis 4, wobei das Gelenkgetriebe (20) einen Vorsprung (9) aufweist, der an dem ersten Arm (5a) befestigt ist und radial nach außen von dem ersten Arm (5a) vorsteht.

6. Intraokularlinse gemäß einem der Ansprüche 1 bis 5, wobei die Haptik (3) in der Richtung parallel zu der optischen Achse (21) des Optikkörpers (2) unterschiedliche Dicken hat.

7. Intraokularlinse gemäß Anspruch 6, wobei in der Richtung parallel zu der optischen Achse (21) des Optikkörpers (2) der erste Arm (5a) dicker als die anderen der Arme (5b bis 5d) ausgebildet ist.

8. Intraokularlinse gemäß Anspruch 6 oder 7, wobei in der Richtung parallel zu der optischen Achse (21) des Optikkörpers (2) in einem Gelenkbereich, in dem das erste Gelenk (6a) angeordnet ist, die Haptik (3) dünner als in den Bereichen der Haptik (3) ist, die an den Gelenkbereich angrenzen.

9. Intraokularlinse gemäß einem der Ansprüche 1 bis 8, wobei das Gelenkgetriebe (20) einen Gelenkgetriebevorsprung (5d) aufweist, der ein erstes Längsende (18), an dem das vierte Gelenk (6d) angeordnet ist, und ein zweites Längsende (19) aufweist und der mit seinem zweiten Längsende (19) an dem Optikkörper (2) befestigt ist.

## Claims

1. Intraocular lens having an optics body (2) and a haptic (3), **characterized in that** the haptic (3) comprises a linkage mechanism (20) having a first arm (5a), which forms the radially outwardly arranged part of the haptic (3), and has a first longitudinal end (11) and a second longitudinal end (12), a first linkage (6a), which is arranged at a position between the first longitudinal end (11) of the first arm (5a) and the second longitudinal end (12) of the first arm (5a) and by means of which the first arm (5a) is pivotably coupled to the optics body (2), a second arm (5b), a third arm (5c), a second linkage (6b), a third linkage (6c) and a fourth linkage (6d), wherein the second linkage (6b) is arranged at the second longitudinal end (12) of the first arm (5a) and at a first longitudinal end (14) of the second arm (5b) and pivotably couples the first arm (5a) to the second arm (5b), the third linkage (6c) is arranged at a second longitudinal end (15) of the second arm (5b) and at a first longitudinal end (16) of the third arm (5c) and pivotably couples the second arm (5b) to the third arm (5c), the fourth linkage (6d) is arranged at a second longitudinal end (17) of the third arm (5c) and pivotably couples the third arm (5c) to the optics body (2), and also at least one of the second linkage (6b), the third linkage (6c) and the fourth linkage (6d) is configured to exert a restoring force when the linkage mechanism (20) is moved out of its rest position, wherein the haptic (3) comprises a support projection (4) which is embodied to be stiffer than the linkage mechanism (20), which is fastened to the optics body (2), which radially outwardly protrudes from the optics body and which has arranged at its radially outer edge the first linkage (6a).

2. Intraocular lens according to Claim 1, wherein the support projection (4) has at least one of the following features so that the support projection is stiffer than the linkage mechanism (20):
- the support projection (4) is designed to be thicker than the linkage mechanism (20) in the direction parallel to the optical axis (21) of the optics body (2),
- the support projection (4) has ribs,
- the support projection (4) is made of a substance that differs from the substance of the linkage mechanism (20),
- the support projection (4) has stronger polymer crosslinking than the linkage mechanism (20),
- the support projection (4) is fibre-reinforced.

3. Intraocular lens according to Claim 1 or 2, wherein the support projection (4) has a cut-out (10), in particular wherein the cut-out (10) is a through-hole.

4. Intraocular lens according to any of Claims 1 to 3, wherein the at least one of the second linkage (6b), the third linkage (6c) and the fourth linkage (6d) is a living hinge, in order to exert the restoring force.

5. Intraocular lens according to any of Claims 1 to 4, wherein the linkage mechanism (20) comprises a projection (9) which is fastened to the first arm (5a) and radially outwardly protrudes from the first arm (5a).

6. Intraocular lens according to any of Claims 1 to 5, wherein the haptic (3) has different thicknesses in the direction parallel to the optical axis (21) of the optics body (2).

7. Intraocular lens according to Claim 6, wherein the first arm (5a) has a thicker embodiment than the other arms (5b to 5d) in the direction parallel to the optical axis (21) of the optics body (2).

8. Intraocular lens according to Claim 6 or 7, wherein, in the direction parallel to the optical axis (21) of the optics body (2), the haptic (3) is thinner in a linkage region, in which the first linkage (6a) is arranged, than in the regions of the haptic (3) which adjoin the linkage region.

9. Intraocular lens according to any of Claims 1 to 8, wherein the linkage mechanism (20) comprises a linkage mechanism projection (5d) which has a first longitudinal end (18), at which the fourth linkage (6d) is arranged, and a second longitudinal end (19) and which is fastened with its second longitudinal end (19) to the optics body (2) .

## Revendications

1. Lentille intraoculaire comprenant un corps optique (2) et un élément haptique (3), **caractérisée en ce que** l'élément haptique (3) comporte un mécanisme articulé (20) qui présente un premier bras (5a) formant la partie radialement extérieure de l'élément haptique (3) et ayant une première extrémité longitudinale (11) et une deuxième extrémité longitudinale (12), une première articulation (6a), qui est disposée à une position située entre la première extrémité longitudinale (11) du premier bras (5a) et la deuxième extrémité longitudinale (12) du premier bras (5a) et au moyen de laquelle le premier bras (5a) est couplé de manière pivotante au corps optique (2), un deuxième bras (5b), un troisième bras (5c), une deuxième articulation (6b), une troisième articulation (6c) et une quatrième articulation (6d), dans laquelle la deuxième articulation (6b) est disposée à la deuxième extrémité longitudinale (12) du premier bras (5a) et à une première extrémité longitudinale (14) du deuxième bras (5b) et couple de manière pivotante le premier bras (5a) au deuxième bras (5b), la troisième articulation (6c) est disposée à une deuxième extrémité longitudinale (15) du deuxième bras (5b) et à une première extrémité longitudinale (16) du troisième bras (5c) et couple de manière pivotante le deuxième bras (5b) au troisième bras (5c), la quatrième articulation (6d) est disposée à une deuxième extrémité longitudinale (17) du troisième bras (5c) et couple de manière pivotante le troisième bras (5c) au corps optique (2), et au moins l'une de la deuxième articulation (6b), de la troisième articulation (6c) et de la quatrième articulation (6d) est conçue pour exercer une force de rappel lorsque le mécanisme articulé (20) est déplacé hors de sa position de repos, dans laquelle l'élément haptique (3) présente une saillie d'appui (4) réalisée plus rigide que le mécanisme articulé (20), qui est fixée au corps optique (2), qui fait saillie radialement vers l'extérieur du corps optique et sur le bord radialement extérieur de laquelle est disposée la première articulation (6a).

2. Lentille intraoculaire selon la revendication 1, dans laquelle la saillie d'appui (4) présente au moins l'une des caractéristiques suivantes, afin que la saillie d'appui soit plus rigide que le mécanisme articulé (20) :
- la saillie d'appui (4) est réalisée plus épaisse que le mécanisme articulé (20) dans la direction parallèle à l'axe optique (21) du corps optique (2),
- la saillie d'appui (4) présente des nervures,
- la saillie d'appui (4) est constituée d'un matériau qui est différent du matériau du mécanisme articulé (20),
- la saillie d'appui (4) présente une réticulation polymère plus forte que le mécanisme articulé (20),
- la saillie d'appui (4) est renforcée par des fibres.

3. Lentille intraoculaire selon la revendication 1 ou 2, dans laquelle la saillie d'appui (4) présente un évidement (10), en particulier dans laquelle l'évidement (10) est un trou traversant.

4. Lentille intraoculaire selon l'une des revendications 1 à 3, dans laquelle l'au moins une de la deuxième articulation (6b), de la troisième articulation (6c) et de la quatrième articulation (6d) est une articulation à film destinée à exercer la force de rappel.

5. Lentille intraoculaire selon l'une des revendications 1 à 4, dans laquelle le mécanisme articulé (20) présente une saillie (9) fixée au premier bras (5a) et faisant saillie radialement vers l'extérieur du premier bras (5a).

6. Lentille intraoculaire selon l'une des revendications 1 à 5, dans laquelle, dans la direction parallèle à l'axe optique (21) du corps optique (2), l'élément haptique (3) présente des épaisseurs différentes.

7. Lentille intraoculaire selon la revendication 6, dans laquelle, dans la direction parallèle à l'axe optique (21) du corps optique (2), le premier bras (5a) est plus épais que les autres des bras (5b à 5d).

8. Lentille intraoculaire selon la revendication 6 ou 7, dans laquelle, dans la direction parallèle à l'axe optique (21) du corps optique (2), dans une zone d'articulation dans laquelle est disposée la première articulation (6a), l'élément haptique (3) est plus mince que dans les zones de l'élément haptique (3) qui sont adjacentes à la zone d'articulation.

9. Lentille intraoculaire selon l'une des revendications 1 à 8, dans laquelle le mécanisme articulé (20) présente une saillie de mécanisme articulé (5d) qui présente une première extrémité longitudinale (18), sur laquelle est disposée la quatrième articulation (6d), et une deuxième extrémité longitudinale (19), et qui est fixée par sa deuxième extrémité longitudinale (19) au corps optique (2).
